# EUROPEAN PATENT APPLICATION

(11) **EP 3 878 940 A1**
(43) Date of publication of application: **15.09.2021**
(21) Application number: 19883192.7
(22) Date of filing: 01.11.2019
(51) Int. Cl.: C12M 3/00

(54) **CELL CULTURING CHIP AND PRODUCTION METHOD THEREFOR**

(30) Priority: 07.11.2018 JP 2018210069
(71) Applicant: Ushio Denki Kabushiki Kaisha, Tokyo 100-8150 (JP); Kyoto University, Kyoto-shi, Kyoto 606-8501 (JP)
(72) Inventor: YAMANAKA, Makoto, Tokyo 100-8150 (JP); KAMEI, Kenichiro, Kyoto-shi, Kyoto 606-8501 (JP)
(74) Representative: Tomerius, Isabel
(86) International application number: PCT/JP2019/043109
(87) International publication number: WO 2020/095849

(57) **Abstract**

A cell culture chip includes a plate having a first surface, a first opening formed inside the plate and having one end exposed on the first surface, a second opening formed inside the plate and at a location different from the first opening and having one end exposed on the first surface, a hollow connecting section communicating with the other end of the first opening and the other end of the second opening, and a water repellent section with water repellent treatment being provided at least in the vicinity of the first opening or the second opening on the first surface of the plate.

## Description

### TECHNICAL FIELD

The present invention relates to a cell culture chip and a manufacturing method thereof.

### BACKGROUND ART

Microplates have conventionally been regarded as laboratory instruments that enable observation and inspection of a large number of samples at one time. (Refer, for example, to Patent Literature 1) A microplate is a flat plate instrument with multiple microfluidic channels, and is capable of cultivating and inspecting cells, microorganisms, or the like to be inspected under different conditions in each microfluidic channel. The microplate can be used to readily compare the inspected objects in a large number of microfluidic channels at one time.

### CITATION LIST

### PATENT LITERATURE

Patent Literature 1: International Publication No.2016/158233

### SUMMARY OF INVENTION

### Technical Problem

Methods of supplying solution to microfluidic channels include continuous supply of solution using a pump and tubing, and batch supply of a fixed volume of solution using a micropipette or similar device. However, the method using a pump and tube requires the pump and tube to be placed around the chip, thus increasing the overall size of the chip and complicating the wiring of the power supply. Hence, from the viewpoint of supplying the solution to the microfluidic channels in a simple method, a method of supplying the solution into the microfluidic channels using a micropipette or the like is preferable.

Incidentally, cell culture chips containing microfluidic channels with small volume (capacity) have a narrow interval between its inlet and outlet for a culture medium. Such cell culture chips can also have an array of microfluidic channels due to its small capacity of each microfluidic channel. In this case, the interval between the inlet of one microfluidic channel A and the inlet or outlet of the adjacent microfluidic channel B is also narrow.

Under such a situation, when solution X is introduced into the inlet of a certain microfluidic channel A using a micropipette, the solution X may spread wet around the inlet. As mentioned above, since the interval between the inlet and outlet of the same microfluidic channel A and the interval between adjacent microfluidic channels are narrow, the solution X that has spread wet may enter the outlet or the adjacent microfluidic channels, resulting in cross-contamination (so-called contamination). If this event occurs, it may fail to correctly evaluate the cultured cells or the like.

In view of the above issue, it is an object of the present invention to provide a cell culture chip including microfluidic channels that is unlikely to cause cross contamination during the injection of a culture medium.

### Solution to the Problem

The cell culture chip according to the present invention includes a plate having a first surface, a first opening formed inside the plate and having one end exposed on the first surface, a second opening formed inside the plate and at a location different from the first opening and having one end exposed on the first surface, a hollow connecting section communicating with the other end of the first opening and the other end of the second opening, and a water repellent section with water repellent treatment being provided at least in the vicinity of the first opening or the second opening on the first surface of the plate.

According to the cell culture chip, cells can be cultured in the connecting section by injecting a culture medium containing cells from the first opening or the second opening using, for example, a micropipette. Since water repellent treatment is applied at least to the vicinity of the first opening or the second opening on the first surface of the plate, even if the culture medium happens to flow at the area around the openings during injecting the culture medium into the openings with the water repellent treatment, it will stay in droplets near the openings on the first surface, preventing the culture medium from flowing outside of the openings. This suppresses the culture medium from flowing into the adjacent culture spaces (connecting sections), even in a case of culturing multiple cells in the adjacent culture spaces (connecting sections) using the cell culture chip.

In addition, as mentioned above, the culture medium that leaks into the water repellent section forms water droplets due to the water repellent treatment applied to the water repellent section. Thus, it enables the leaked medium to readily return to the openings (first opening and second openings). Variation in the amount of the culture medium supplied to the culture space may influence the state of the cultured cells; however, the configuration described above can supply a predetermined amount of the culture medium to each culture space, thus improving the accuracy of the experiment.

In the present specification, the "vicinity" of the first opening or the second opening may refer to an area from the periphery of each opening on the first surface to a position that is distant at a length of at least 0.5 mm. The water repellent section is not necessarily formed to completely cover the periphery of the first opening or the second opening, and a non-water repellent section may be formed in a part of the area outside the periphery. More specifically, it is preferable that the water repellent section be provided in an area that accounts for between 70% and 100% of the area enclosed from the outer periphery of the first opening or the second opening to a position that is distant at a length equivalent to the radius of the opening (or the radius of the inscribed circle if the opening is non-circular). It is more preferable that the water repellent section be provided to include an area between 70% and 100% of the outer periphery of the opening.

The water repellent section preferably has water resistance, chemical durability, and biocompatibility, in addition to water repellent property. For example, the water repellent section can include a material containing a fluorine group polymer. Specifically, the water repellent section can be formed by applying a fluoropolymer solution with a stamp or an inkjet.

The cell culture chip may have a volume of space that extends from the one end of the first opening to the one end of the second opening through the connecting section, to be 100 µL or less.

In such a configuration, the space for culturing cells is constituted by a micro space. As described above, this configuration, in which water repellent treatment is applied at least to the vicinity of the first opening or the second opening on the first surface of the plate, prevents the culture medium from flowing out over the first surface of the plate, when a small amount of the culture medium is injected into the first opening or the second opening.

The one end of the first opening and the one end of the second opening formed on the first surface may both have an inner diameter of 5 mm or less. The separation distance between the one end of the first opening and the one end of the second opening formed on the first surface may be 20 mm or less.

On a single cell culture chip, a plurality of culture spaces including the first openings, the connecting sections, and the second openings may be formed. In this case, the separation distance between each opening (first opening) of the adjacent culture spaces is preferably 10 mm or less. This configuration enables the simultaneous cultivation of multiple cells under different environments, resulting in achieving a high density and a high throughput cultivation. Furthermore, the water repellent sections provided around each opening reduces a risk of the culture medium mixing into adjacent culture spaces, thus eliminating the problem of cross-contamination.

The present invention is a method for manufacturing the cell culture chip described above, the method includes
a step (a) for fabricating a first substrate having at least two through holes penetrating from one surface to the other surface and a hole communicating between the through holes, and a second substrate being a flat shape;
a step (b) for applying water repellent treatment to the vicinity of an area where at least one of the through holes is exposed on the one surface of the first substrate; and
a step (c) for bonding the other surface of the first substrate with the second substrate to fabricate the plate.

With the above method, a cell culture chip that has a first opening, a second opening, and a connecting section communicating the openings, and also that has a water repellent section with water repellent treatment on the surface in the vicinity of at least one of the openings, is fabricated using the two through holes and the hole.

The present invention enables a cell culture chip that is unlikely to cause cross contamination during the injection of a culture medium.

### BRIEF DESCRIPTION OF DRAWINGS

FIG. 1 is a schematic perspective view of a cell culture chip illustrating a configuration of an embodiment.
FIG. 2 is a schematic plan view of the cell culture chip viewed from a first substrate side.
FIG. 3 is a schematic cross-sectional view of the cell culture chip taken along line X1 - X1 in FIG. 2.
FIG. 4 is a schematic cross-sectional view of the cell culture chip in a state in which a culture medium has been injected into the cell culture chip in accordance with FIG. 3.
FIG. 5 is a schematic cross-sectional view of the cell culture chip to describe its dimensions.
Fig. 6 is a schematic plan view of a cell culture chip in a state in which a culture medium has been injected into the cell culture chip that does not have a water repellent section.
FIG. 7 is a schematic cross-sectional view of the cell culture chip in a state in which a culture medium has been injected into the cell culture chip that does not have a water repellent section.
FIG. 8A is a schematic plan view of a cell culture chip having a plurality of culture chambers.
FIG. 8B is a schematic cross-sectional view of the cell culture chip taken along line A1-A1 in FIG. 8A, illustrating a state in which a culture medium has been injected into a plurality of culture chambers.
FIG. 9 is a process cross-sectional view schematically illustrating the manufacturing method of a cell culture chip.
FIG. 10 is a schematic cross-sectional view of a cell culture chip illustrating a configuration of another embodiment.
FIG. 11 is a schematic plan view illustrating an example of the shape of the water repellent section.
FIG. 12A is a schematic plan view of a cell culture chip according to another embodiment viewed from a first substrate side.
FIG. 12B is a schematic plan view of a cell culture chip according to another embodiment viewed from a first substrate side.

### DESCRIPTION OF EMBODIMENTS

The cell culture chip and its manufacturing method in accordance with the present invention will now be described with reference to the drawings. It is noted that the following drawings are just schematically illustrated. In other words, the dimensional ratios on the drawings do not necessarily match the actual dimensional ratios, and the dimensional ratios between each drawing do not necessarily match either.

### [Configuration]

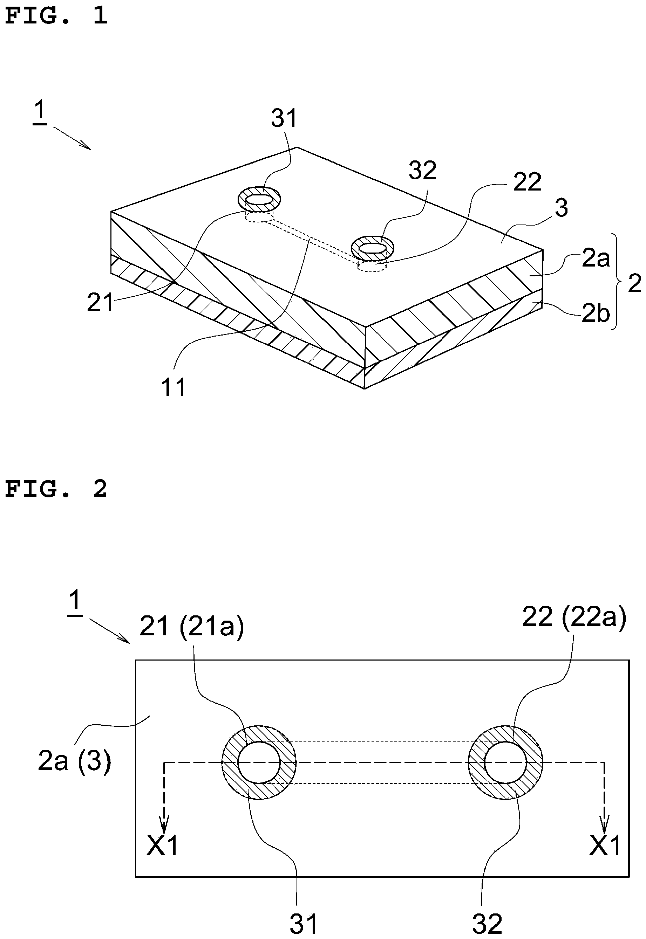

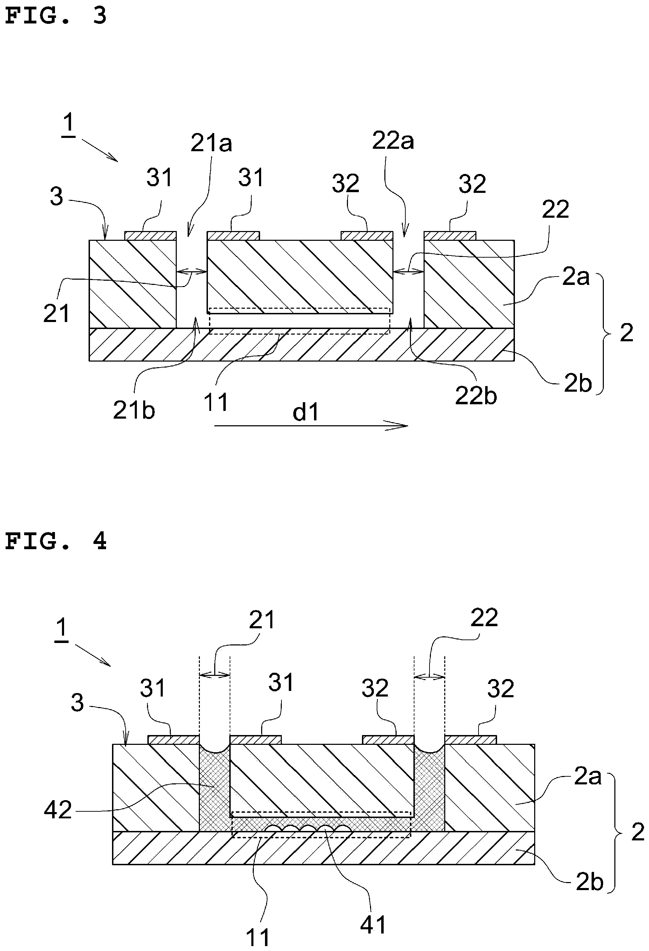

FIG. 1 is a schematic perspective view of a cell culture chip illustrating a configuration of an embodiment. The cell culture chip 1 is provided with a plate 2 that consists of a first substrate 2a and a second substrate 2b. FIG. 2 is a schematic plan view of a cell culture chip 1 viewed from the first substrate 2a side. FIG. 3 is a schematic cross-sectional view of the cell culture chip 1 taken along line X1 -X1 in FIG. 2.

In the present embodiment, the cell culture chip 1 is provided with the plate 2 that consists of the first substrate 2a and the second substrate 2b. Among the plate 2, two through holes are formed in the first substrate 2a at separated positions, and one surface of these through holes is in contact with the second substrate 2b to form a first opening 21 and a second opening 22. In other words, when the cell culture chip 1 is viewed from the first substrate 2a side, one end 21a of the first opening 21 and one end 22a of the second opening 22 are exposed, as shown in FIG. 2. Among the surfaces of the first substrate 2a, the surface 3 where the ends (21a, 22a) of each opening (21, 22) are exposed corresponds to the "first surface" as shown in FIG. 2. Hereinafter, this surface is suitably referred to as "first surface 3".

The first substrate 2a has a narrow tubular recess on the side of the second substrate 2b surface, and the area enclosed by this recess and the second substrate 2b constitutes the connecting section 11. The connecting section 11 is composed of a hollow channel that communicates with the end 21b that is opposite to the end 21a of the first opening 21, and with the end 22b that is opposite to the end 22a of the second opening 22. In the present embodiment, the connecting section 11 constitutes a space for culturing cells (a culture chamber).

In other words, the connecting section 11, which constitutes a culture chamber, consists of a narrow tubular space that is surrounded by the walls of the plate 2 and that extends in the direction from the first opening 21 to the second opening 22 defined as a longitudinal direction d1 (see FIG. 3). For example, in FIG. 3, by injecting a culture medium 42 containing cells 41 into the end 21a side of the first opening 21, the cells 41 are cultured in the connecting section 11 that constitutes the culture chamber (see also FIG. 4).

The first surface 3 of the first substrate 2a has an area (31, 32) with water repellent treatment at least in the vicinity of an area where the ends (21a, 22a) of each opening (21, 22) are formed. These areas are suitably referred to as "water repellent section 31" and "water repellent section 32".

The water repellent section 31 may be formed at least in the vicinity of the end 21a of the opening 21 on the first surface 3 of the first substrate 2a. Similarly, the water repellent section 32 may be formed at least in the vicinity of the end 22a of the opening 22 on the first surface 3 of the first substrate 2a. The term "the vicinity of the end 21a of the opening 21" refers to an area from the outer periphery (outer edge) of the end 21a of the opening 21 to an outward position that is distant at 50% of the inner diameter of the end 21a. Similarly, the term "the vicinity of the end 22a of the opening 22" refers to the area from the outer periphery (outer edge) of the end 22a of the opening 22 to an outward position that is distant at 50% of the inner diameter of the end 22a.

The water repellent section 31 and the water repellent section 32 can be formed with any material or method, as long as they have functionality of repelling liquid. A hydrophobic material such as a fluoropolymer or silicone, for example, can be applied to a predetermined area on the first surface 3 of the first substrate 2a. A microstructure having the functionality of water repellent property due to the Lotus effect may also be provided.

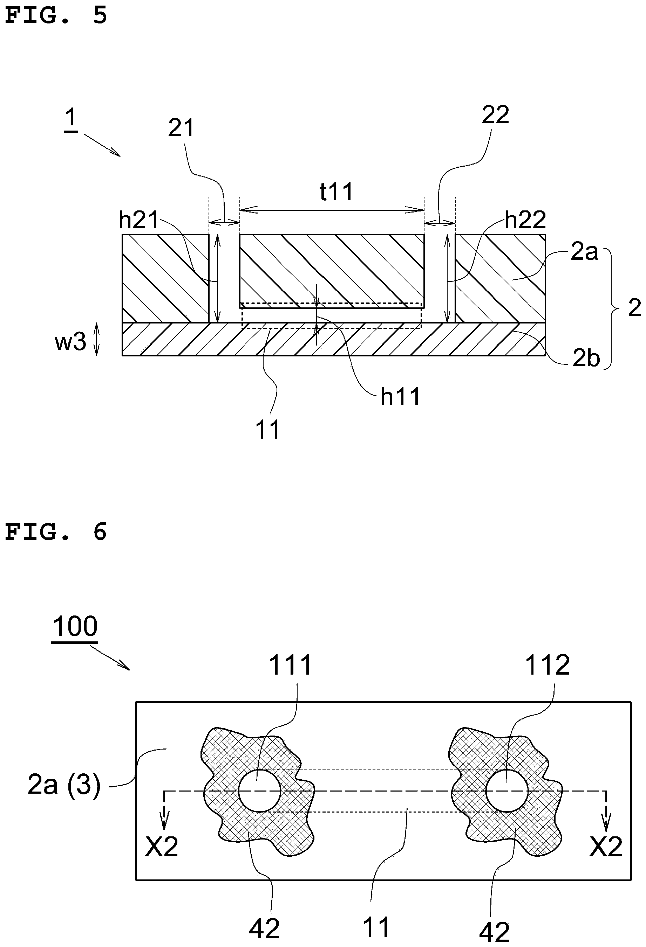

An example of the dimensions is as follows (see FIG. 5). The second substrate 2b has a height (thickness) w3 of about 1 mm, preferably 100 µm or more and 2 mm or less. The first opening 21 has a height h21 of about 3 mm, and the second opening 22 has a height h22 of about 3 mm. The connecting section 11 (culture chamber) has a height h11 of about 300 µm, and preferably 200 µm or more and 500 µm or less. The connecting section 11 (culture chamber) has a length t11 of about 9 mm in the longitudinal direction. In the present embodiment, the length t11 approximately corresponds to a separation distance between the end 21a of the first opening 21 and the end 22a of the second opening 22.

As shown in FIG. 2, the end 21a of the first opening 21 and the end 22a of the second opening 22 both have an inner diameter of about 2 mm. The end 21a and the end 22a preferably have a diameter (inner diameter) of the circumscribed circle (the circle itself if the end is circular) of 5 mm or less, and more preferably 3 mm or less.

The first opening 21 does not have to have a uniform inner diameter from the end 21a toward the end 21b, and may have an area with a different inner diameter. The similar manner applies to the second opening 22.

The volume of the space that extends from the end 21a of the first opening 21 to the end 22a of the second opening 22 through the connecting section 11 is 100 mm³ (100 µL) or less, and more preferably 10 mm³ (10 µL).

The first substrate 2a and the second substrate 2b that constitute the plate 2 are preferably made of a substantially non-porous material. The term "substantially non-porous" refers to a state in which the apparent surface area of the medium is approximately the actual surface area thereof. Examples of materials that constitute the above non-porous materials include inorganic materials such as glass and silicon, or resin materials such as polymethyl methacrylate (PMMA), polycarbonate (PC), cyclo-olefin copolymer (COC), cyclo-olefin polymer (COP), and polystyrene (PS). The examples may include a combination of two or more of these resin materials. Configuring the plate 2 with the materials described above allows bioactive substances released from cells 41 that are cultured in the connecting section 11 to be returned to the cells 41 side again, while preventing them from being absorbed into the plate 2 that constitutes the wall of the connecting section 11.

The first substrate 2a and the second substrate 2b, which constitute the plate 2, are preferably composed of a material transparent to light. In the case that the plate 2 is made of the resin material described above, the cells 41 can be observed from outside the cell culture chip 1.

As described above, the inner diameter of each opening (21, 22) and the diameter of the ends (21a, 22a) that constitute the opening surface of each opening (21, 22) are extremely small according to the present embodiment. The volume of the space that is composed of the openings (21, 22) and the connecting section 11 is also extremely small. When the culture medium 42 containing cells 41 is injected into such a microspace, a method using an instrument that can supply only a very small amount of liquid in a fixed quantity, such as a micropipette, can be used.

In the present embodiment, the water repellent sections (31, 32) are provided in the vicinity of the ends (21a, 22a) of each opening (21, 22). Hence, for example, when the culture medium 42 is injected using a micropipette into the first opening 21, even if this culture medium 42 leaks out to a location outside the first opening 21 on the first surface 3 of the first substrate 2a, the leaked medium is repelled on the first surface 3, thus avoiding further leakage outward (see FIG. 4). In FIG. 4, each water repellent section (31, 32) is shown as having a height; however, this is merely for convenience of illustration. Each water repellent section (31, 32) may actually be formed as a very thin film on the first surface 3.

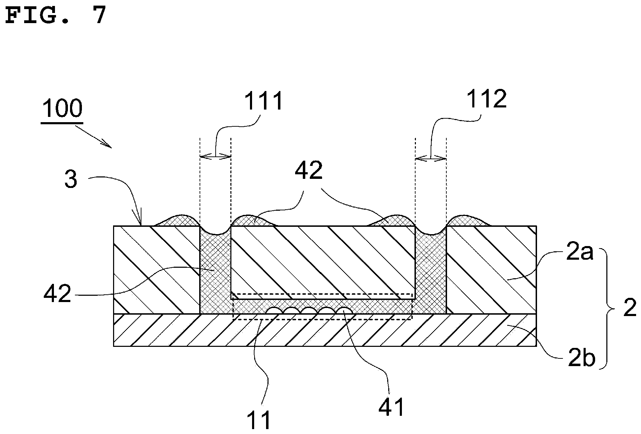

In contrast, as shown in FIGS. 6 and 7, in the case of the cell culture chip 100 that does not have the water repellent sections (31, 32), when the culture medium 42 that is injected into the first opening 21 overflows onto the upper surface of the first surface 3, then the overflowed medium flows over the first surface 3 as it is. FIG. 6 corresponds to a plan view of the cell culture chip viewed from the first substrate 2a side in accordance with FIG. 2, and FIG. 7 corresponds to a cross-sectional view taken along line X2-X2 in FIG. 6. FIG. 6 and 7 schematically illustrate a case where the amount of injected culture medium 42 is large enough to cause the culture medium 42 to overflow at the second opening 22 as well.

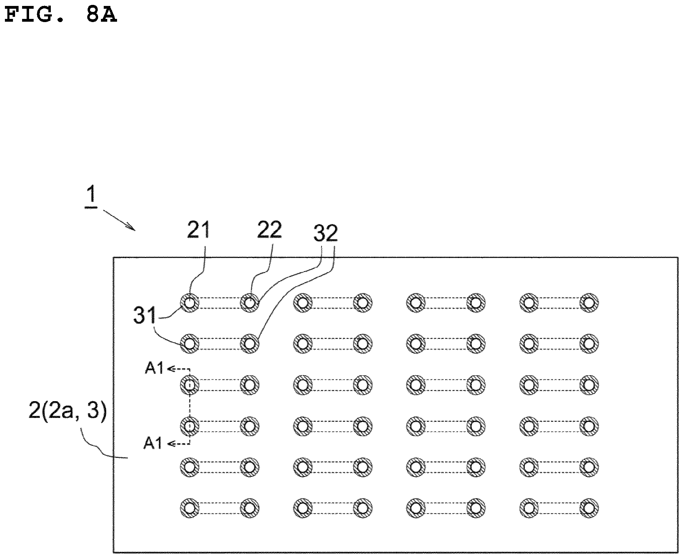

As described above, the channel composed of the first opening 21, the connecting section 11, and the second opening 22 is extremely small in size. Hence, the plurality of channels are expected to be formed independently of each other in a single plate 2, as illustrated in FIG. 8A. This configuration allows a plurality of cells 41 to be cultured in parallel, thus improving the efficiency of experiment and evaluation. It is noted that the number and arrangement of the culture spaces shown in FIG. 8A are merely an example. For example, FIG. 8A illustratively shows a case where a plurality of culture spaces are arranged in the row direction and the column direction; however, one culture space may be formed in one direction.

As shown in FIGS. 6 and 7, in the case that no water repellent treatment is applied on the first surface 3 in the vicinity of the openings (111, 112), the culture medium 42 that is injected into the openings111 may flow over the first surface 3 of the first substrate 2a and flow into the adjacent culture chamber through the openings (111, 112), which communicate with the connecting section 11 constituting the adjacent culture chamber. If this event occurs, bioactive substances released from cells 41 that are cultured in one culture chamber (connecting section 11) may flow into another culture chamber (connecting section 11) and have an influence on cells 41 that are cultured in the another culture chamber. This situation may prevent the correct evaluation of cultured cells.

In contrast, since the cell culture chip 1 of the present embodiment is provided with the water repellent sections (31, 32) in the vicinity of the openings (21, 22) on the first surface 3, even if the culture medium 42 flows out of the openings (21, 22), the overflowed medium forms water droplets to stay in the sections, preventing it from flowing into the adjacent culture chamber. As described above, in the case of supplying the culture medium 42 into the connecting section 11, which constitutes the culture chamber of the cell culture chip 1, the culture medium 42 needs to be injected through the openings (21, 22) having small diameters; then, the culture medium 42 may overflow outside the openings (21, 22). If the culture medium 42 overflows outside the openings (21, 22), the overflowed medium may spread to the surrounding area. However, as described above, since the water repellent sections (31, 32) are provided in the vicinity of the openings (21, 22) on the first surface 3, even if the culture medium 42 overflows from the openings (21, 22), the overflowed medium forms droplets to stay in the sections, preventing it from spreading to the surrounding area.

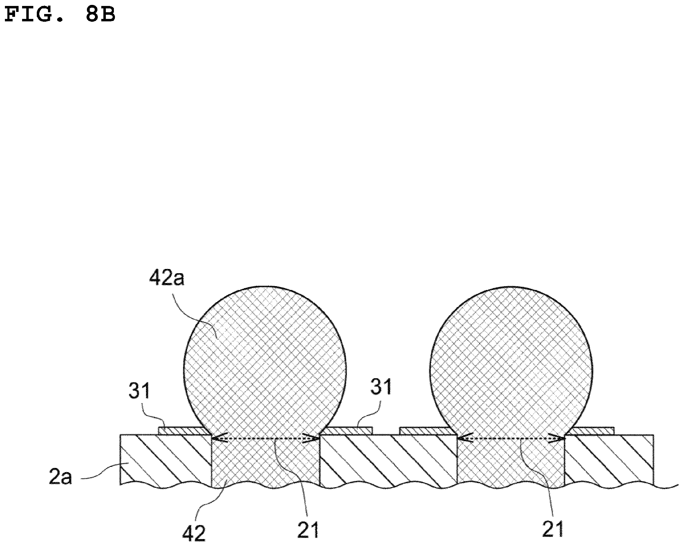

FIG. 8B is a schematic cross-sectional view of the cell culture chip 1 taken along line A1 -A1 in FIG. 8A, illustrating a state in which a culture medium 42 has been injected into a cell culture chip 1. Since the water repellent section 31 is provided around the periphery of the opening 21, the culture medium 42 forms a water droplet (water droplet 42a) inside the water repellent section 31, preventing the culture medium 42 from flowing into the adjacent opening 21. For example, in the case that the opening 21 has an inner diameter of 2 mm, the water droplet 42a formed on the upper surface of the opening 21 has an approximate diameter of 2.8 mm or more to 3.8 mm or less.

### [Manufacturing Method]

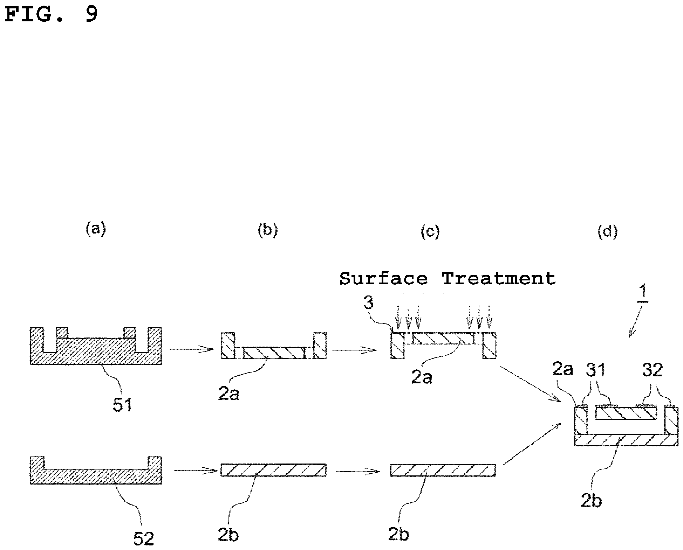

An example of the manufacturing method of the cell culture chip 1 will be explained with reference to FIG. 9. As shown in FIG. 9(a), molds (51, 52) having predetermined shapes are prepared. The mold 51 is a mold for the first substrate 2a, and the mold 52 is a mold for the second substrate 2b. The mold 51 has a shape that corresponds to the openings (21a, 21b) of the first substrate 2a.

Next, as shown in FIG. 9(b), these molds (51, 52) are used to perform injection molding with the materials described above (e.g., resin material) to fabricate the first substrate 2a and the second substrate 2b (step (a)). Then, as shown in FIG. 9(c), water repellent treatment is applied to a predetermined area on the first surface 3 side of the first substrate 2a (step (b)). Specifically, as described above, fluoropolymer resin is applied to the predetermined area with an ink-jet method or a stamp method. The method of surface treatment for water repellent is not limited to the above method; however, it is preferable that the method do not contaminate the through holes and the hole that are provided in the first substrate 2a.

Then, as shown in FIG. 9(d), the surface of the first substrate 2a opposite the first surface 3 is bonded with the second substrate 2b (step (c)). During the bonding step, it is preferable that the method do not contaminate the openings (21, 22) and the channel inside the connecting section 11, which are to be formed after the bonding step. Specifically, it is preferable to perform this step without using adhesives. Examples of the method may include a method in which the bonding surfaces of both substrates (2a, 2b) are irradiated with vacuum ultraviolet light for surface treatment, and then pressurized and heated while the bonding surfaces are in contact with each other.

It may be possible to perform steps (b) and (c) without using the mold in step (a); instead, with using the first substrate 2a and the second substrate 2b that have been prepared in advance and have shapes as shown in FIG. 9(b). In step (c), the surface treatment may be applied to the entire outer surface of each substrate (2a, 2b).

### [Another Embodiment]

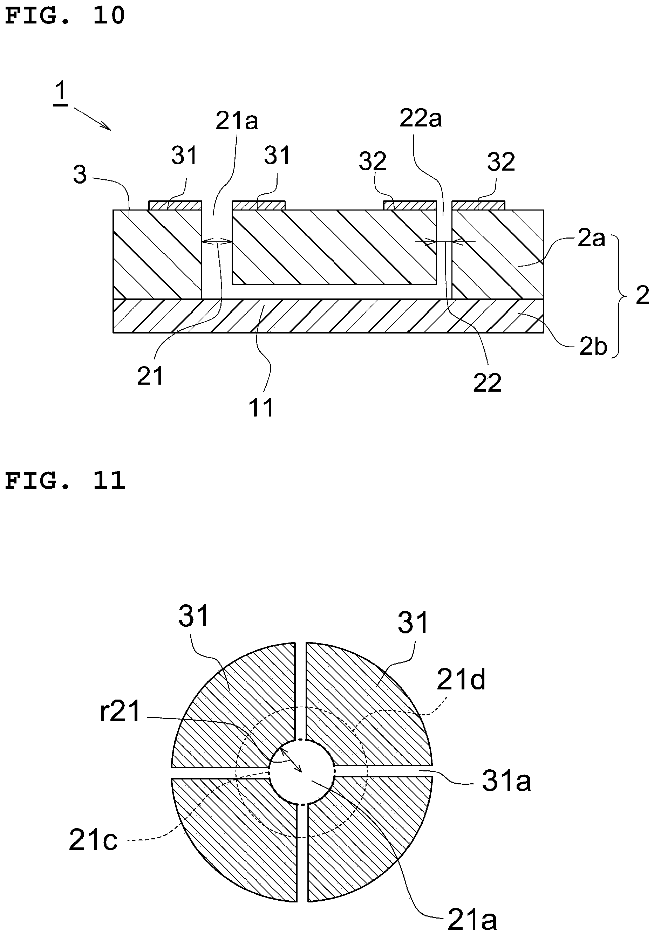

Hereinafter, another embodiment is explained.
<1 > In the above embodiment, it is explained that the first surface 3 of the first substrate 2a has sections with water repellent treatment (water repellent section 31, water repellent section 32) at least in the vicinity of the area where the ends (21a, 22a) of each opening (21, 22) are formed. However, the water repellent treatment may be applied to the entire first surface 3a of the first substrate 2a.
   The water repellent treatment may be applied to the vicinity of only one opening of the first surface 3 of the first substrate 2a, for example, the vicinity of the area where the end 21a of the first opening 21 is formed. In this case, the culture medium 42 may be injected into the opening with the water repellent treatment (in this case, the first opening 21).
<2> As shown in FIG. 10, in the cell culture chip 1, the first opening 21 may have an inner diameter that is different from that of the second opening 22. In this case, the culture medium 42 can be injected into the first opening 21 that has a larger diameter, and the culture medium 42 can be taken out from the second opening 22 that has a smaller diameter. In this case, the water repellent section 31 may be provided only in the vicinity of the end 21a of the first opening 21, which is the side where the culture medium 42 is injected, and no water repellent section 32 may be provided in the vicinity of the end 22a of the second opening 22.
<3> In the above embodiment, the case is explained such that the connecting section 11 that constitutes the culture chamber, and each opening (21, 22) have the common bottom surface, which is the top surface of the second substrate 2b; however, this is merely one example. It is preferable that the connecting section 11 and each opening (21, 22) have a common bottom surface, in terms of enabling the manufacturing process of the cell culture chip 1 to be simplified and the cell culture chip 1 to be made extremely small in size.
<4> The embodiment described above with reference to FIG. 2 explains a case where the water repellent section 31 is provided to surround the end 21a of the first opening 21; however, the water repellent section 31 does not necessarily need to be formed to completely surround the end 21a of the first opening 21. For example, as shown in FIG. 11, non-water repellent sections 31a may be formed in part of the area around the periphery of the end 21a of the first opening 21. In the step (b) described above, particularly in the case that the water repellent section 31 is formed by the spray deposition of a water repellent material while masking an area other than those where the water repellent section 31 is to be formed, the non-water repellent section 31a is formed on the first surface 3 of the first substrate 2a at a location facing the bridge for holding the mask.

The water repellent section 31 is preferably provided in an area that accounts for 70% or more of the area enclosed from the outer periphery 21c of the first opening 21 to a position that is distant at a length equivalent to the radius r21 of the first opening 21 (i.e., the area bounded by the outer periphery 21c and the virtual circle 21d). Furthermore, it is more preferable that the water repellent section 31 be provided to include an area of 70% or more of the outer periphery 21c of the first opening 21. The similar manner may apply to the water repellent section 32 provided on the second opening 22.

While FIG. 11 illustrates a case where the non-water repellent sections 31a are provided at multiple locations outside the first opening 21, they may be provided at a single location. Any number and any shape of the non-water repellent sections 31a can be formed.

The water repellent section 31 does not necessarily have a uniform width from the periphery of the end 21a of the first opening 21. The similar manner may apply to the case where a non-water repellent section 31a is formed.

<5> The above embodiment describes a cell culture chip 1 in which the pair of openings (21, 22) are communicated with the connecting section 11 that constitutes a culture chamber; however, in the cell culture chip 1 of the present invention, the number of openings (21, 22) that are communicated with the connecting section 11 is not limited.

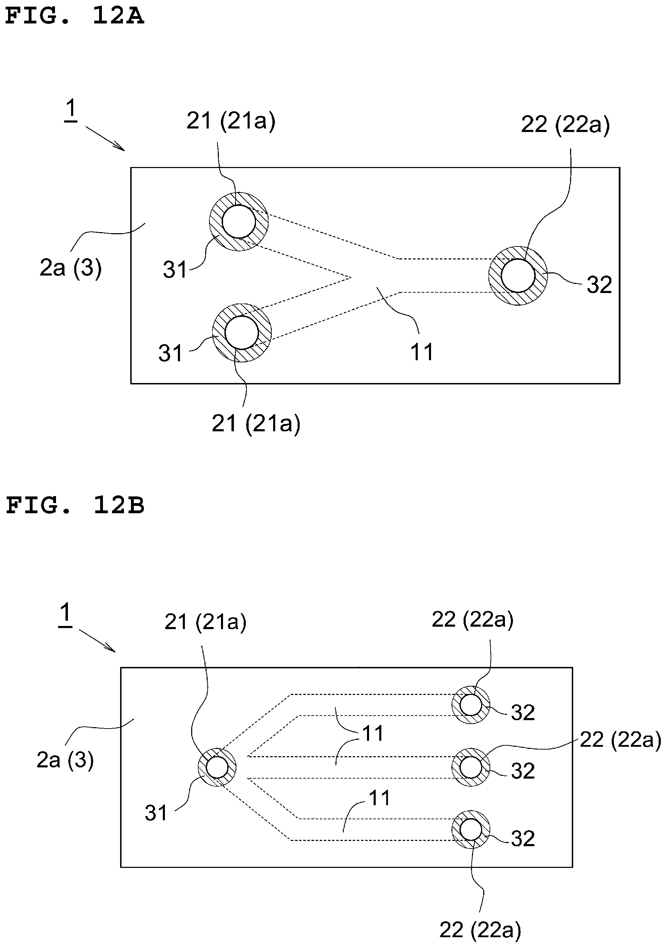

FIGS. 12A and 12B are schematic plan views of a cell culture chip according to another embodiment viewed from the first substrate 2a side in accordance with FIG. 2. The cell culture chip 1 shown in FIG. 12A has two first openings 21 and one second opening 22, and each opening (21, 22) is communicated with a connecting section 11. The cell culture chip 1 shown in FIG. 12B has one first opening 21 and three second openings 22, and three connecting sections 11 are provided to communicate with the first opening 21 and the respective second openings 22.

On both of the cell culture chips 1 shown in FIGS. 12A and 12B, the water repellent sections (31, 32) are formed in the vicinity of the openings (21, 22). As described above, the water repellent sections (31, 32) may be formed only in the vicinity of either of the first opening 21 and the second opening 22. In the case of a plurality of the first openings 21 and the second openings 22, the water repellent sections (31, 32) may be formed only in the vicinity of one or more of the openings (21, 22).

### REFERENCE SIGNS LIST

- 1: cell culture chip
- 2: plate
- 2a: first substrate
- 2b: second substrate
- 3: first surface
- 11: connecting section (culture chamber)
- 21: first opening
- 21a, 21b: end of first opening
- 21c: outer periphery of first opening
- 21d: virtual circle
- 22: second opening
- 22a, 22b: end of second opening
- 31: water repellent section
- 31a: non-water repellent section
- 32: water repellent section
- 41: cell
- 42: culture medium
- 42a: water droplet of culture medium
- 51, 52: mold
- 100: cell culture chip without water repellent section
- 111, 112: opening provided in cell culture chip 100

## Claims

1. A cell culture chip comprising:
a plate having a first surface;
a first opening formed inside the plate and having one end exposed on the first surface;
a second opening formed inside the plate and at a location different from the first opening, the second opening having one end exposed on the first surface; and
a hollow connecting section communicating with the other end of the first opening and the other end of the second opening,
wherein a water repellent section with water repellent treatment is provided at least in the vicinity of the first opening or the second opening on the first surface of the plate.

2. The cell culture chip according to claim 1, wherein the water repellent section includes a material containing a fluorine group polymer.

3. The cell culture chip according to claim 1 or 2, wherein a volume of space extending from the one end of the first opening to the one end of the second opening through the connecting section, is 100 µL or less.

4. The cell culture chip according to claim 3, wherein the one end of the first opening and the one end of the second opening formed on the first surface both have an inner diameter of 5 mm or less.

5. The cell culture chip according to claim 3 or 4, wherein a separation distance between the one end of the first opening and the one end of the second opening formed on the first surface is 20 mm or less.

6. A method for manufacturing the cell culture chip according to claim 1, the method comprising:
a step (a) for fabricating a first substrate having at least two through holes penetrating from one surface to the other surface and a hole communicating between the through holes, and a second substrate being a flat shape;
a step (b) for applying water repellent treatment to the vicinity of an area where at least one of the through holes is exposed on the one surface of the first substrate; and
a step (c) for bonding the other surface of the first substrate with the second substrate to fabricate the plate.
